(19) Europäisches Patentamt
European Patent Office
Office européen des brevets

(11) EP 1 093 788 A1

(12) DEMANDE DE BREVET EUROPEEN

(43) Date de publication:
**25.04.2001 Bulletin 2001/17**

(51) Int Cl.[7]: **A61K 7/13**

(21) Numéro de dépôt: **00402778.5**

(22) Date de dépôt: **09.10.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **21.10.1999 FR 9913148**

(71) Demandeur: **L'OREAL**
**75008 Paris (FR)**

(72) Inventeurs:
- **Pastore, Florent**
  **92500 Rueil Malmaison (FR)**
- **Lagrange, Alain**
  **77700 Coupvray (FR)**

(74) Mandataire: **Fevrier, Murielle**
**L'Oreal,**
**DPI,**
**6 Rue Sincholle**
**92585 Clichy Cedex (FR)**

# (54) Composition de teinture d'oxydation des fibres kératiniques et procédé de teinture mettant en oeuvre cette composition

(57) L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation hétérocyclique choisie parmi les pyrazoles, certaines pyrimidines et les pyrazolopyrimidines, et au moins un coupleur choisi parmi des dérivés 2-substitués d'amino-5-alkylphénols, ainsi que le procédé de teinture mettant en oeuvre cette composition.

EP 1 093 788 A1

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation hétérocyclique choisie parmi les pyrazoles, certaines pyrimidines et les pyrazolopyrimidines, et au moins un coupleur choisi parmi des dérivés 2-substitués d'amino-5-alkylphénols, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet JP-2521636, des compositions pour la teinture d'oxydation des fibres kératiniques contenant, à titre de coupleurs des dérivés 2-substitués d'amino-5-alkylphénols, en association avec une ou plusieurs bases d'oxydation pouvant par exemple être choisies parmi certaines paraphénylènediamines, le para-aminophénol, le para-méthylaminophénol, l'orthoaminophénol, ou bien encore certaines bases d'oxydation hétérocycliques comme la 2,5-diaminopyridine ou la tétraaminopyridine. Cependant, les colorations obtenues en mettant en oeuvre ces compositions ne sont pas toujours assez puissantes, chromatiques ou résistantes aux différentes agressions que peuvent subir les cheveux.

Or, la Demanderesse vient maintenant de découvrir qu'il est possible d'obtenir de nouvelles teintures, capables de conduire à des colorations aux nuances variées, chromatiques, puissantes, esthétiques, peu sélectives et résistant bien aux diverses agressions que peuvent subir les fibres, en associant au moins une base d'oxydation hétérocyclique choisie parmi les pyrazoles, certaines pyrimidines et les pyrazolopyrimidines, et au moins un coupleur choisi parmi les dérivés 2-substitués d'amino-5-alkylphénols de formule (I) définie ci-après et leurs sels d'addition avec un acide.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation hétérocyclique choisie parmi :

  **i**) les pyrazoles,
  **ii**) les pyrimidines choisies parmi les diamino-pyrimidines, les diamino-pyrimidines mono- ou di-hydroxylées, les triamino-pyrimidines, et les triamino-pyrimidines mono-hydroxylées,
  **iii**) les pyrazolopyrimidines,

  et leurs sels d'addition avec un acide ;

- et au moins un coupleur choisi parmi les dérivés 2-substitués d'amino-5-alkylphénols de formule (I) suivante, et leurs sels d'addition avec un acide :

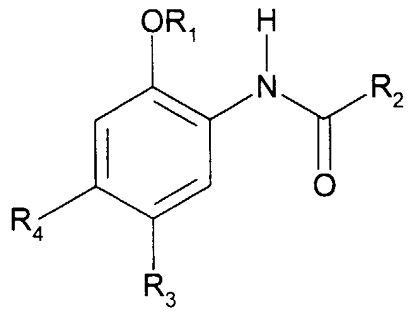

(I)

dans laquelle :

- $R_1$ représente un atome d'hydrogène, ou un radical acyle en $C_2$-$C_5$ substitué ou non substitué ;
- $R_2$ représente un atome d'hydrogène, un radical alkyle en $C_1$-$C_4$ substitué ou non substitué, un radical alcoxy en $C_1$-$C_4$ substitué ou non substitué, ou un radical amino substitué ou non substitué ;
- $R_3$ représente un atome d'hydrogène ou d'halogène, un radical alcoxy en $C_1$-$C_4$, ou un radical monohydroxyalcoxy en $C_1$-$C_4$ ; et
- $R_4$ représente un radical alkyle en $C_1$-$C_4$.

La composition tinctoriale conforme à l'invention conduit à des colorations dans des nuances variées, chromatiques, puissantes, esthétiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Parmi les pyrazoles pouvant être utilisés à titre de base d'oxydation dans la composition tinctoriale conforme à l'invention, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 1-(β-hydroxyéthyl) pyrazole, le 4,5-diamino 1-(β-hydroxyéthyl) pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les pyrimidines particulières pouvant être utilisées à titre de base d'oxydation dans la composition tinctoriale conforme à l'invention, on peut notamment citer la 4-hydroxy 2,5,6-triaminopyrimidine, la 2-hydroxy 4,5,6-triaminopyrimidine, la 6-hydroxy 2,4,5-triaminopyrimidine, la 2,4-dihydroxy 5,6-diaminopyrimidine, la 2,5,6-triaminopyrimidine, et leurs sels d'addition avec un acide. Ces composés sont décrits par exemple dans les brevets allemand DE 2 359 399 ou japonais JP 88-169 571 et JP 91-10659 ou dans la demande de brevet WO 96/15765.

Parmi les pyrazolopyrimidines pouvant être utilisées à titre de base d'oxydation dans la composition tinctoriale conforme à l'invention, on peut notamment citer celles qui sont mentionnées dans la demande de brevet FR-A-2 750 048 et parmi lesquelles figurent par exemple la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs sels d'addition avec un acide, et leurs formes tautomères, lorsqu'il existe un équilibre tautomérique.

Les dérivés 2-substitués d'amino-5-alkylphénols de formule (I) utilisés à titre de coupleurs dans la composition tinctoriale conforme à l'invention, leurs intermédiaires de synthèse et leurs procédés de préparation sont décrits la

demande de brevet JP-2521636, dont le contenu fait partie intégrante de la présente demande.

Parmi les radicaux alkyle en $C_1$-$C_4$ pouvant être représentés par les radicaux $R_2$ et $R_4$ des composés de formule (I) définie ci-dessus, on peut par exemple mentionner les radicaux méthyle, éthyle, n-propyle, isopropyle, n-butyle, sec-butyle et tert-butyle.

Parmi les radicaux alcoxy en $C_1$-$C_4$ pouvant être représentés par les radicaux $R_2$ et $R_3$ des composés de formule (I) définie ci-dessus, on peut par exemple mentionner les radicaux méthoxy, éthoxy, n-propoxy, isopropoxy, n-butoxy, sec-butoxy et tert-butoxy.

Parmi les radicaux acyle en $C_2$-$C_5$ pouvant être représentés par le radical $R_1$ des composés de formule (I) définie ci-dessus, on peut par exemple mentionner les radicaux acétyle, propanoyle, 2,2-diméthylpropanoyl, 3-méthylpropanoyle, butanoyle, 2-méthylbutanoyle, et pentanoyle.

Parmi les atomes d'halogène pouvant être représentés par le radical $R_3$ des composés de formule (I) définie ci-dessus, on peut par exemple mentionner le fluor, le chlore, le brome et l'iode.

Dans les composés de formule (I) ci-dessus, le radical $R_4$ représente de préférence un radical méthyle ou éthyle ; le radical $R_2$ représente de préférence un radical méthyle, éthyle, méthoxy ou amino ; le radical $R_1$ représente de préférence un atome d'hydrogène, un radical acétyle ou propanoyle ; le radical $R_3$ représente de préférence un atome d'hydrogène ou un radical méthoxy.

A titre de substituants des radicaux alkyle en $C_1$-$C_4$ et alcoxy en $C_1$-$C_4$ et acyle en $C_2$-$C_5$ mentionnés précédemment, on peut notamment citer les atomes d'halogène tels que le fluor, le chlore, le brome et l'iode ; les radicaux alcoxy en $C_1$-$C_4$ tels que méthoxy, éthoxy, n-propoxy, isopropoxy, n-butoxy, sec-butoxy et tert-butoxy ; les radicaux alkanoyloxy en $C_2$-$C_5$ tels que acétoxy, propanoyloxy, 2,2-diméthylpropanoyloxy, 3-méthylpropanoyloxy, butanoyloxy, 2-méthylbutanoyloxy, et pentanoyloxy ; le radical hydroxyle ; le groupement amino ; les radicaux amino protégés par un groupement protecteur ; le radical carboxyle ; les radicaux alcoxycarbonyle en $C_2$-$C_5$ tels que méthoxycarbonyle, éthoxycarbonyle, n-propoxycarbonyle, isopropoxycarbonyle, n-butoxycarbonyle, sec-butoxycarbonyle, tert-butoxycarbonyle. Parmi ces substituants, les radicaux méthoxy et hydroxyle sont préférés.

A titre de groupements protecteurs des radicaux amino mentionnés ci-dessus, on peut citer, à titre d'exemple, les groupes tert-butoxycarbonyle et benzyloxycarbonyle.

A titre de substituants des radicaux amino mentionnés précédemment pour R2, on peut notamment citer les radicaux alkyle en $C_1$-$C_4$ non substitués ou substitués par un ou plusieurs radicaux hydroxyle. Parmi ces substituants, le radical hydroxyéthyle est préféré.

Parmi les dérivés 2-substitués d'amino-5-alkylphénols de formule (I) définie ci-dessus, on peut plus particulièrement citer :

- le N-(2-hydroxy-4-méthylphényl)-formamide,
- le N-(2-hydroxy-4-méthylphényl)-méthylcarbamate,
- la N-(2-hydroxy-4-méthylphényl)-urée,
- la N-(2-hydroxy-4-méthylphényl)-N'-(2-hydroxyéthyl)-urée,
- le N-(2-hydroxy-4-méthylphényl)-acétamide,
- le N-(2-hydroxy-4-méthylphényl)-chloroacétamide,
- le N-(2-hydroxy-4-méthylphényl)-acétoxyacétamide,
- le N-(2-hydroxy-4-méthylphényl)-hydroxyacétamide,
- le N-(2-hydroxy-4-méthylphényl)-méthoxyacétamide,
- le N-(2-hydroxy-4-méthylphényl)-aminoacétamide,
- le N-(2-hydroxy-4-méthylphényl)-propionamide,
- le N-(2-hydroxy-4-méthylphényl)-2-hydroxypropionamide,
- le N-(2-hydroxy-4-méthylphényl)-3-aminopropionamide,
- le N-(2-hydroxy-4-méthylphényl)-butanamide,
- le N-(2-hydroxy-4-méthylphényl)-4-chlorobutanamide,
- le N-(2-hydroxy-4-méthylphényl)-succinamide,
- le N-(2-hydroxy-4-isopropylphényl)-acétamide,
- le 2'-acétamide-5'-méthylphényl-acétate,
- le 2'-propionamide-5'-méthylphényl-propionate,
- le N-(2-hydroxy-5-méthoxy-4-méthylphényl)-acétamide,

et leurs sels d'addition avec un acide.

La ou les bases d'oxydation hétérocycliques conformes à l'invention représentent de préférence de 0,0005 à 12 % en poids environ du poids total de la composition tinctoriale conforme à l'invention, et encore plus préférentiellement de 0,005 à 6 % en poids environ de ce poids.

Le ou les dérivés 2-substitués d'amino-5-alkylphénols de formule (I) et/ou le ou leurs sels d'addition avec un

acide représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut, en outre, renfermer une ou plusieurs bases d'oxydation additionnelles différentes des bases d'oxydation hétérocycliques conformes à l'invention et/ou un ou plusieurs coupleurs additionnels différents des coupleurs de formule (I) conforme à l'invention et/ou un ou plusieurs colorants directs.

Parmi ces coupleurs additionnels, on peut notamment citer les métaphénylènediamines, les méta-aminophénols, les métadiphénols et les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Ces coupleurs additionnels sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'a-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

D'une manière générale, les sels d'addition avec un acide utilisables dans le cadre des compositions tinctoriales de l'invention, (bases d'oxydation et coupleurs), sont notamment choisis parmi les chlorhydrates, les bromhydrates, les sulfates, les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (II) suivante :

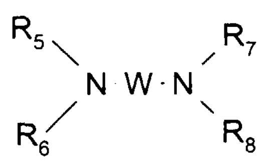

(II)

dans laquelle W est un reste propylène éventuellement substitué par un groupement hydroxyle ou un radical alkyle en $C_1$-$C_4$ ; $R_5$, $R_6$, $R_7$ et $R_8$, identiques ou différents, représentent un atome d'hydrogène, un radical alkyle en $C_1$-$C_4$ ou hydroxyalkyle en $C_1$-$C_4$.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques tels que par exemple des gommes de guar non-ioniques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, et les enzymes parmi lesquelles on peut citer les peroxydases, les oxydo-réductases à 2 électrons telles que les uricases, et les oxygénases à 4 électrons comme les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**EXEMPLES 1 A 3 DE TEINTURE EN MILIEU ALCALIN**

On a préparé les compositions tinctoriales, conformes à l'invention, suivantes :

| EXEMPLE | 1 | 2 | 3 |
|---|---|---|---|
| Dichlorhydrate de 4,5-diamino 1-éthyl 3-méthyl pyrazole (base d'oxydation hétérocyclique) | $3.10^{-3}$ mole | - | - |
| Sulfate de 2,4,5-triamino 6-hydroxy pyrimidine (base d'oxydation hétérocyclique) | - | $3.10^{-3}$ mole | - |
| Dichlorhydrate de pyrazolo-[1,5-a]-pyrimidine-3,7-diamine (base d'oxydation hétérocyclique) | - | - | $3.10^{-3}$ mole |

(suite)

| EXEMPLE | 1 | 2 | 3 |
|---|---|---|---|
| N-(2-hydroxy-4-méthylphényl)-acétamide (coupleur de formule (I) conforme à l'invention) | $3.10^{-3}$ mole | $3.10^{-3}$ mole | $3.10^{-3}$ mole |
| Support de teinture commun | (*) | (*) | (*) |
| Eau déminéralisée q.s.p. | 100 g | 100 g | 100g |

(*) Support de teinture commun :
- Alkyl $C_8$-$C_{10}$ polyglucoside en solution aqueuse à 60%, vendu sous la dénomination ORAMIX CG 110 ® par la société SEPPIC 5,4 g
- Ethanol 18,0 g
- Alcool benzylique 1,8 g
- Polyéthylène glycol 400 2,7 g
- Sel pentasodique de l'acide diéthylène triamine pentacétique en solution aqueuse à 40%, vendu sous la dénomination DISSOLUINE D-40 ® par la société AKZO 1,08 g
- Métabisulfite de sodium 0,205 g
- Ammoniaque à 20,5% de $NH_3$ 10,0 g

Au moment de l'emploi, on a mélangé poids pour poids chacune des compositions tinctoriales décrites ci-dessus avec une solution de peroxyde d'hydrogène à 20 volumes (6% en poids).

Chacun des mélanges ainsi réalisé a été appliqué pendant 30 minutes sur des mèches de cheveux gris naturels à 90 % de blancs. Les mèches ont ensuite été rincées, lavées avec un shampooing standard, rincées à nouveau puis séchées.

Les cheveux ont été teints dans une nuance figurant dans le tableau ci-après :

| EXEMPLE | NUANCE OBTENUE |
|---|---|
| 1 | Violet moyen |
| 2 | Blond clair doré |
| 3 | Pourpre soutenu |

**EXEMPLES DE TEINTURE 4 à 6 COMPARATIFS**

On a préparé les compositions tinctoriales suivantes :

| EXEMPLE | 4 (**) | 5 | 6 |
|---|---|---|---|
| Paraphénylènediamine (base d'oxydation ne faisant pas partie de l'invention) | $3.10^{-3}$ mole | - | - |
| Sulfate de 2,4,5-triamino 6-hydroxy pyrimidine (base d'oxydation hétérocyclique) | - | $3.10^{-3}$ mole | - |
| Dichlorhydrate de pyrazolo-[1,5-a]-pyrimidine-3,7-diamine (base d'oxydation hétérocyclique) | - | - | $3.10^{-3}$ mole |
| N-(2-hydroxy-4-méthylphényl)-acétamide (coupleur de formule (I) conforme à l'invention) | $3.10^{-3}$ mole | $3.10^{-3}$ mole | $3.10^{-3}$ mole |
| Support de teinture commun | (*) | (*) | (*) |
| Eau déminéralisée q.s.p. | 100 g | 100 g | 100 g |

(**): composition ne faisant pas partie de l'invention.

(*) Support de teinture commun :
Il est identique à celui utilisé ci-dessus dans les exemples 1 à 3.

Chacune des compositions tinctoriales ci-dessus a ensuite été appliquée sur des mèches de cheveux gris naturels à 90% de blancs dans les conditions décrites ci-dessus pour les exemples 1 à 3.

La couleur des mèches ainsi teintes a ensuite été évaluée dans le système L*.a*.b*., au moyen d'un spectrophotomètre MINOLTA CM2002 ®, afin de calculer la chromaticité, (luminosité), des colorations obtenues.

Dans le système L* a* b*, les trois paramètres désignent respectivement l'intensité (L*), la nuance (a*) et la

saturation (b*).

Selon ce système, plus la valeur de L* est élevée, plus la couleur est claire ou peu intense. Inversement, plus la valeur de L* est faible, plus la couleur est foncée ou très intense.

a* et b* indiquent deux axes de couleurs, a* indique l'axe de couleur vert/rouge et b* l'axe de couleur bleu/jaune. Des valeurs proches de zéro pour a* et b* correspondent à des nuances grises.

La chromaticité de la coloration (C) peut être calculée en appliquant l'équation suivante :

$$C = \sqrt{a^{*2} + b^{*2}}$$

Dans cette équation, C représente la Chromaticité de la mèche teinte, $a^{*2}$, et $b^{*2}$ représentent respectivement la nuance et la saturation de la mèche teinte élevées au carré.

Plus la valeur de C est importante, plus la Chromaticité, (ou luminosité), de la coloration est importante.

Les résultats obtenus figurent dans le tableau ci-après :

| EXEMPLE | a* | b* | C |
|---|---|---|---|
| **4 (**)** | 0.64 | -7.94 | **8.0** |
| **5** | 2.77 | 12 | **12.3** |
| **6** | 11.2 | -5.69 | **12.6** |

(**): Exemple ne faisant pas partie de l'invention

Ces résultats montrent que les compositions tinctoriales des exemples 5 et 6 conformes à l'invention, c'est à dire contenant l'association d'une base d'oxydation hétérocyclique conforme à l'invention et du N-(2-hydroxy-4-méthylphényl)-acétamide à titre de coupleur, conduisent à des colorations nettement plus chromatiques que celle obtenue avec la composition de l'exemple 4 ne faisant pas partie de l'invention puisque dans cette composition la base d'oxydation hétérocyclique a été remplacée par une base d'oxydation ne faisant pas partie de l'invention, à savoir la paraphénylènediamine.

**EXEMPLES 7 ET 8 COMPARATIFS**

On a préparé les compositions tinctoriales suivantes :

| EXEMPLE | 7 | 8 (**) |
|---|---|---|
| Dichlorhydrate de 4,5-diamino 1-éthyl 3-méthyl pyrazole (base d'oxydation hétérocyclique) | $3.10^{-3}$ mole | - |
| Paraphénylènediamine (base d'oxydation ne faisant pas partie de l'invention) | - | $3.10^{-3}$ mole |
| N-(2-hydroxy-4-méthylphényl)-acétamide (coupleur de formule (I) conforme à l'invention) | $3.10^{-3}$ mole | $3.10^{-3}$ mole |
| Support de teinture commun | (*) | (*) |
| Eau déminéralisée q.s.p. | 100 g | 100 g |

(**): composition ne faisant pas partie de l'invention.

(*) Support de teinture commun :
Il est identique à celui utilisé ci-dessus dans les exemples 1 à 3.

Chacune des compositions tinctoriales ci-dessus a ensuite été appliquée sur des mèches de cheveux gris permanentés à 90% de blancs dans les conditions décrites ci-dessus pour les exemples 1 à 3.

La couleur des mèches ainsi teintes a ensuite été évaluée dans le système L*. a*. b*., au moyen d'un spectrophotomètre MINOLTA CM 2002 ®, afin de calculer la chromaticité, (luminosité), des colorations obtenues, de la même façon que pour les exemples 4 à 6 ci-dessus.

Les résultats obtenus figurent dans le tableau ci-après :

| EXEMPLE | a* | b* | C |
|---|---|---|---|
| **7** | 5.99 | -16.22 | **17.3** |
| **8(**)** | 0.81 | -11.58 | **11.6** |

(**): Exemple ne faisant pas partie de l'invention

Ces résultats montrent que la composition tinctoriale de l'exemple 7 conforme à l'invention, c'est à dire contenant l'association d'une base d'oxydation hétérocyclique conforme à l'invention et du N-(2-hydroxy-4-méthylphényl)-acétamide à titre de coupleur, conduit à des colorations nettement plus chromatiques que celle obtenue avec la composition de l'exemple 8 ne faisant pas partie de l'invention puisque dans cette composition la base d'oxydation hétérocyclique a été remplacée par une base d'oxydation ne faisant pas partie de l'invention, à savoir la paraphénylènediamine.

## Revendications

**1.** Composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation hétérocyclique choisie parmi :

  **i**) les pyrazoles,
  **ii**) les pyrimidines choisies parmi les diamino-pyrimidines, les diamino-pyrimidines mono- ou di-hydroxylées, les triamino-pyrimidines, et les triamino-pyrimidines mono-hydroxylées,
  **iii**) les pyrazolopyrimidines,

  et leurs sels d'addition avec un acide ;

- et au moins un coupleur choisi parmi les dérivés 2-substitués d'amino-5-alkylphénols de formule (I) suivante, et leurs sels d'addition avec un acide :

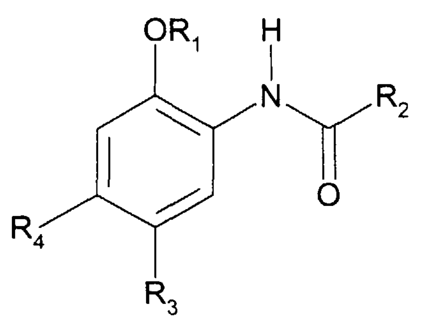

(I)

dans laquelle :

- $R_1$ représente un atome d'hydrogène, ou un radical acyle en $C_2$-$C_5$ substitué ou non substitué ;
- $R_2$ représente un atome d'hydrogène, un radical alkyle en $C_1$-$C_4$ substitué ou non substitué, un radical alcoxy en $C_1$-$C_4$ substitué ou non substitué, ou un radical amino substitué ou non substitué ;
- $R_3$ représente un atome d'hydrogène ou d'halogène, un radical alcoxy en $C_1$-$C_4$, ou un radical monohydroxyalcoxy en $C_1$-$C_4$ ; et
- $R_4$ représente un radical alkyle en $C_1$-$C_4$.

**2.** Composition selon la revendication 1, caractérisée par le fait que les pyrazoles sont choisis parmi le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 1-(β-hydroxyéthyl) pyrazole, le 4,5-diamino 1-(β-hydroxyéthyl) pyrazole, le 4,5-diamino 3-méthyl 1-phényl pyrazole, le 4,5-diamino 1-méthyl 3-phényl pyrazole, le 4-amino 1,3-diméthyl 5-hydrazino

pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

**3.** Composition selon la revendication 1, caractérisée par le fait que les pyrimidines sont choisies parmi la 4-hydroxy 2,5,6-triaminopyrimidine, la 2-hydroxy 4,5,6-triaminopyrimidine, la 6-hydroxy 2,4,5-triaminopyrimidine, la 2,4-dihydroxy 5,6-diaminopyrimidine, la 2,5,6-triaminopyrimidine, et leurs sels d'addition avec un acide.

**4.** Composition selon la revendication 1, caractérisée par le fait que les pyrazolopyrimidines sont choisies parmi la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, et leurs sels d'addition avec un acide, et leurs formes tautomères, lorsqu'il existe un équilibre tautomérique.

**5.** Composition selon l'une quelconque des revendications précédentes, caractérisée par le fait que les dérivés 2-substitués d'amino-5-alkylphénols de formule (I) sont choisis parmi :

- le N-(2-hydroxy-4-méthylphényl)-formamide,
- le N-(2-hydroxy-4-méthylphényl)-méthylcarbamate,
- la N-(2-hydroxy-4-méthylphényl)-urée,
- la N-(2-hydroxy-4-méthylphényl)-N'-(2-hydroxyéthyl)-urée,
- le N-(2-hydroxy-4-méthylphényl)-acétamide,
- le N-(2-hydroxy-4-méthylphényl)-chloroacétamide,
- le N-(2-hydroxy-4-méthylphényl)-acétoxyacétamide,
- le N-(2-hydroxy-4-méthylphényl)-hydroxyacétamide,
- le N-(2-hydroxy-4-méthylphényl)-méthoxyacétamide,
- le N-(2-hydroxy-4-méthylphényl)-aminoacétamide,
- le N-(2-hydroxy-4-méthylphényl)-propionamide,
- le N-(2-hydroxy-4-méthylphényl)-2-hydroxypropionamide,
- le N-(2-hydroxy-4-méthylphényl)-3-aminopropionamide,
- le N-(2-hydroxy-4-méthylphényl)-butanamide,
- le N-(2-hydroxy-4-méthylphényl)-4-chlorobutanamide,
- le N-(2-hydroxy-4-méthylphényl)-succinamide,
- le N-(2-hydroxy-4-isopropylphényl)-acétamide,
- le 2'-acétamide-5'-méthylphényl-acétate,
- le 2'-propionamide-5'-méthylphényl-propionate,
- le N-(2-hydroxy-5-méthoxy-4-méthylphényl)-acétamide,

et leurs sels d'addition avec un acide.

**6.** Composition selon l'une quelconque des revendications précédentes, caractérisée par le fait que la ou les bases d'oxydation hétérocycliques représentent de 0,0005 à 12 % en poids du poids total de la composition tinctoriale.

**7.** Composition selon la revendication 6, caractérisée par le fait que la ou les bases d'oxydation hétérocycliques représentent de 0,005 à 6 % en poids du poids total de la composition tinctoriale.

**8.** Composition selon l'une quelconque des revendications précédentes, caractérisée par le fait que le ou les dérivés 2-substitués d'amino-5-alkylphénols de formule (I) et/ou le ou leurs sels d'addition avec un acide représentent de 0,0005 à 12 % en poids du poids total de la composition tinctoriale.

**9.** Composition selon la revendication 8, caractérisée par le fait que le ou les dérivés 2-substitués d'amino-5-alkylphénols de formule (I) et/ou le ou leurs sels d'addition avec un acide représentent de 0,005 à 6 % en poids du poids total de la composition tinctoriale.

**10.** Composition selon l'une quelconque des revendications précédentes, caractérisée par le fait que les sels d'addition avec un acide sont choisis parmi les chlorhydrates, les bromhydrates, les sulfates, les tartrates, les lactates et les acétates.

**11.** Procédé de teinture des fibres kératiniques et en particulier des fibres kératiniques humaines telles que les cheveux, caractérisé par le fait qu'on applique sur lesdites fibres au moins une composition tinctoriale telle que définie à l'une quelconque des revendications 1 à 10, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**12.** Procédé selon la revendication 11, caractérisé par le fait que l'agent oxydant présent dans la composition oxydante est choisi parmi le peroxyde d'hydrogène, le peroxyde d'urée, les bromates de métaux alcalins, les persels, les peracides, et les enzymes.

**13.** Dispositif à plusieurs compartiments, ou "kit" de teinture à plusieurs compartiments, dont un premier compartiment renferme une composition tinctoriale telle que définie à l'une quelconque des revendications 1 à 10 et un second compartiment renferme une composition oxydante.

Office européen des brevets

# RAPPORT DE RECHERCHE EUROPEENNE

Numéro de la demande
EP 00 40 2778

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 579 204 A (KAO CORP)<br>19 janvier 1994 (1994-01-19)<br>* page 3, ligne 12-24; revendications 1,3 *<br>* page 7, ligne 1-16 *<br>--- | 1-3,11 | A61K7/13 |
| X | DE 196 04 178 A (HENKEL KGAA)<br>7 août 1997 (1997-08-07)<br>* page 2, ligne 52-57; revendication 1 *<br>----- | 1,3,11 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| BERLIN | 12 janvier 2001 | Beyss, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO. EP 00 40 2778

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-01-2001

| Document brevet cité au rapport de recherche | Date de publication | Membre(s) de la famille de brevet(s) | Date de publication |
|---|---|---|---|
| EP 0579204 A | 19-01-1994 | JP 2521636 B | 07-08-1996 |
| | | JP 6080542 A | 22-03-1994 |
| | | US 5334225 A | 02-08-1994 |
| DE 19604178 A | 07-08-1997 | AUCUN | |

EPO FORM P0460

Pour tout renseignement concernant cette annexe : voir Journal Officiel de l'Office européen des brevets, No.12/82